# EUROPEAN PATENT APPLICATION

(11) **EP 4 759 894 A1**
(43) Date of publication of application: **17.06.2026**
(21) Application number: 24851707.0
(22) Date of filing: 31.07.2024
(51) Int. Cl.: C09K 5/10

(54) **COMPOSITION FOR HEAT TRANSFER FLUID, DEVICE HAVING HEAT TRANSFER MECHANISM, AND HEAT TRANSFER METHOD**

(30) Priority: 10.08.2023 JP 2023131648; 19.04.2024 JP 2024068662
(71) Applicant: Daikin Industries, Ltd., Osaka-shi, Osaka 530-0001 (JP)
(72) Inventor: MARI, Daichi, Osaka-Shi, Osaka 530-0001 (JP); SHIRAI, Atsushi, Osaka-Shi, Osaka 530-0001 (JP); MATSUURA, Makoto, Osaka-Shi, Osaka 530-0001 (JP); KUROKI, Yoshichika, Osaka-Shi, Osaka 530-0001 (JP)
(74) Representative: Hoffmann Eitle
(86) International application number: PCT/JP2024/027338
(87) International publication number: WO 2025/033282

(57) **Abstract**

The present invention provides a composition containing a hexafluoropropene trimer represented by C₉F₁₈, wherein the composition is usable as a replacement for a heat transfer fluid containing at least one selected from the group consisting of perfluorotripropylamine, perfluoropolyether, and a methoxytridecafluoroheptene isomer mixture.

## Description

### TECHNICAL FIELD

The present disclosure relates to a composition for heat transfer fluid, a device comprising a heat transfer mechanism, and a heat transfer method.

### BACKGROUND ART

In electric equipment and electronic equipment, thermal management is necessary. For example, during production of semiconductor wafers, the temperature of the process needs to be controlled. It is also necessary to remove excess heat that can be produced in microprocessors, data centers, power electronics, and aircrafts. To solve these challenges, heat transfer fluid is used. Generally, heat transfer fluid of this type desirably has low kinematic viscosity and excellent dielectric properties.

Heat transfer fluid is also used as two-phase immersion cooling fluid, chiller fluid, Rankine cycle working fluid, and the like.

For these purposes, the following products are currently used:
·Perfluorotripropylamine (or tris(heptafluoropropyl)amine, or N,N-bis(heptafluoropropyl)(heptafluoropropyl)amine) [N(CF₂CF₂CF₃)ₙ(CF(CF₃)CF₃)₃₋ₙ (where n is an integer of 0 to 3)], including a product under the trade name "Fluorinert (registered trademark)" (manufactured by 3M) (FC-3283) (PTL 1);
·Perfluoropolyether (PFPE) including products under the trade names GALDEN (registered trademark) "HT135" and GALDEN (registered trademark) "HT110" (manufactured by Solvay), and more specifically tetrafluoroethylene oxide polymers (PTL 2); and
·Methoxytridecafluoroheptene isomer mixtures including a product under the trade name "Opteon SF10" (manufactured by Chemours), and more specifically methyl-perfluoroheptene ether (MPHE) (C₇F₁₃OCH₃) (NPL 1).

### CITATION LIST

### PATENT LITERATURE

| | |
|---|---|
| PTL 1: | Japanese National Patent Publication No. 2016-505882 |
| PTL 2: | WO2010/034698 |

### NON PATENT LITERATURE

NPL 1: Aaron M. Jubb, and four others "Methyl-Perfluoroheptene-Ethers (CH3OC7F13): Measured OH Radical Reaction Rate Coefficients for Several Isomers and Enantiomers and Their Atmospheric Lifetimes and Global Warming Potentials", 2014, Environ. Sci. Technol., 48, 4954-4962

### SUMMARY OF INVENTION

### TECHNICAL PROBLEM

The present disclosure aims at providing a heat transfer fluid that can replace the above-mentioned existing heat transfer fluids.

### SOLUTION TO PROBLEM

The inventors of the present invention have conducted intensive research to achieve the above-mentioned objective, and as a result, have found that a compound represented by C₉F₁₈ is usable as a replacement for the above-mentioned existing heat transfer fluids. Based on this finding, the inventors of the present invention have conducted further investigation and arrived at the present disclosure.

More specifically, the present disclosure includes below-described aspects.

Aspect 1. A composition containing a hexafluoropropene trimer represented by C₉F₁₈, wherein the composition is usable as a replacement for a heat transfer fluid containing at least one selected from the group consisting of perfluorotripropylamine, perfluoropolyether, and a methoxytridecafluoroheptene isomer mixture.

Aspect 2. The composition according to Aspect 1, wherein the hexafluoropropene trimer represented by C₉F₁₈ includes at least one compound selected from the group consisting of compounds represented by formulae (I) to (III) below.

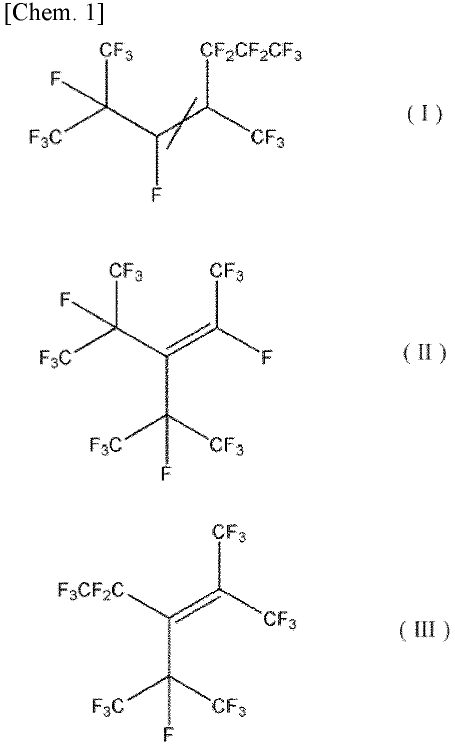

Aspect 3. The composition according to Aspect 2, wherein the hexafluoropropene trimer represented by C₉F₁₈ includes the compound represented by the formula (I) in less than 85 mass% relative to a total of the hexafluoropropene trimer.

Aspect 4. The composition according to any one of Aspects 1 to 3, wherein the composition satisfies conditions (i) to (vi) below:
(i) having a boiling point that is 80% or more of a boiling point of the heat transfer fluid;
(ii) having a pour point that is equal to or less than a pour point of the heat transfer fluid;
(iii) having a kinematic viscosity that is 200% or less of a kinematic viscosity of the heat transfer fluid; and
(iv) being compatible with the heat transfer fluid at a freely-selected proportion.

Aspect 5. A composition for heat transfer fluid, comprising:
a hexafluoropropene trimer represented by C₉F₁₈; and
at least one selected from the group consisting of perfluorotripropylamine, perfluoropolyether, and a methoxytridecafluoroheptene isomer mixture.

Aspect 6. The composition for heat transfer fluid according to Aspect 5, wherein the hexafluoropropene trimer represented by C₉F₁₈ includes at least one compound selected from the group consisting of compounds represented by formulae (I) to (III) below.

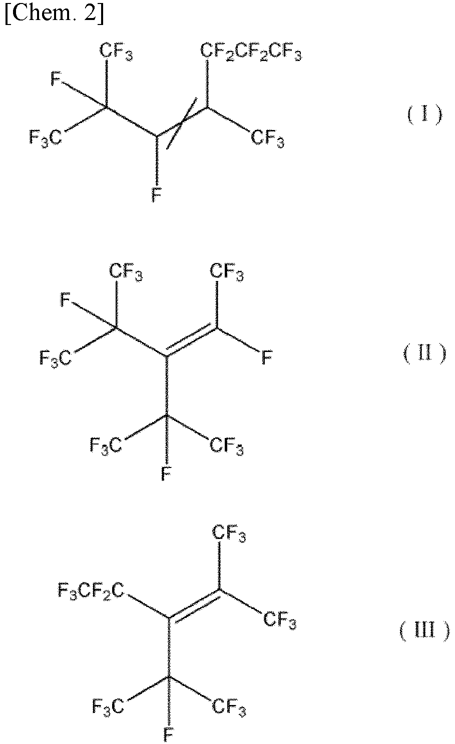

Aspect 7. The composition for heat transfer fluid according to Aspect 6, wherein the hexafluoropropene trimer represented by C₉F₁₈ includes the compound represented by the formula (I) in less than 85 mass% relative to a total of the hexafluoropropene trimer represented by C₉F₁₈.

Aspect 8. The composition for heat transfer fluid according to Aspect 6, wherein the compound includes the compound represented by the formula (I) in 85 mass% or more relative to a total of the hexafluoropropene trimer represented by C₉F₁₈.

Aspect 9. A device comprising:
a device; and
a heat transfer mechanism that transfers heat to the device or from the device by using the composition according to any one of Aspects 1 to 4 or the composition for heat transfer fluid according to any one of Aspects 5 to 8 as a heat transfer fluid,
wherein the heat transfer mechanism is designed to transfer heat by using a heat transfer fluid containing at least one selected from the group consisting of perfluorotripropylamine, perfluoropolyether, and a methoxytridecafluoroheptene isomer mixture.

Aspect 10. Use of a hexafluoropropene trimer represented by C₉F₁₈ as a replacement for a heat transfer fluid containing at least one selected from the group consisting of perfluorotripropylamine, perfluoropolyether, and a methoxytridecafluoroheptene isomer mixture.

Aspect 11. A heat transfer method implementable in a device comprising a heat transfer mechanism designed to transfer heat by using a heat transfer fluid containing at least one selected from the group consisting of perfluorotripropylamine, perfluoropolyether, and a methoxytridecafluoroheptene isomer mixture, the heat transfer method comprising:
transferring heat to the device or from the device by operating the heat transfer mechanism with the use of the composition according to any one of Aspects 1 to 4 or the composition for heat transfer fluid according to any one of Aspects 5 to 8 as a replacement for the heat transfer fluid.

### ADVANTAGEOUS EFFECTS OF INVENTION

The present disclosure makes it possible to provide a heat transfer fluid that can replace a heat transfer fluid containing at least one selected from the group consisting of perfluorotripropylamine, perfluoropolyether, and a methoxytridecafluoroheptene isomer mixture.

### DESCRIPTION OF EMBODIMENTS

Herein, the term "contain" conceptually encompasses "comprise", "consist essentially of", and "consist of". Moreover, herein, a numerical range expressed as "(from) A to B" means not less than A and not more than B.

### 1. Composition Containing Compound Represented by C₉F₁₈

### (1) Heat Transfer Fluid

A composition according to the present disclosure contains a heat transfer fluid compound, and the heat transfer fluid compound includes a compound represented by C₉F₁₈.

The compound represented by C₉F₁₈ is preferably a hexafluoropropene (HFP) trimer.

The hexafluoropropene trimer is not limited and may be selected from a wide variety of known ones represented by C₉F₁₈.

Specific examples of the hexafluoropropene trimer include compounds represented by formulae (I) to (III) below.

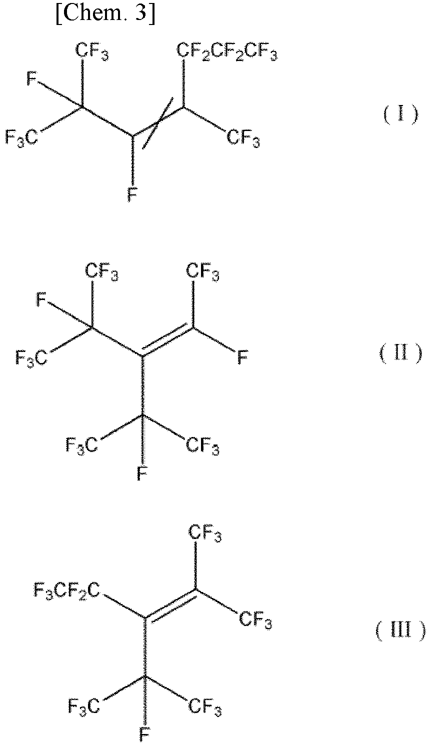

In the present disclosure, the compound represented by the formula (I) includes both E- and Z-diastereomers unless otherwise specified.

The hexafluoropropene trimer included in the compound represented by C₉F₁₈ according to the present disclosure may be only one of the compounds represented by the formulae (I) to (III), or a mixture of two or three of them.

When the HFP trimer is a mixture, the proportion of the compound represented by the formula (I) in the entire mixture (namely, in the total amount of the compounds represented by the formulae (I), (II), and (III) contained in the mixture) is preferably 1 mass% or more, more preferably 10 mass% or more, yet further preferably 30 mass% or more, particularly preferably 40 mass% or more, particularly preferably 45 mass% or more, further particularly preferably 50 mass% or more, relative to the entire HFP trimer. Moreover, the compound represented by the formula (I) in the total of the HFP trimer is preferably 99 mass% or less, more preferably 90 mass% or less, further preferably 85 mass% or less (or less than 85 mass%), yet further preferably 80 mass% or less, particularly preferably 70 mass% or less, most preferably 60 mass% or less. For example, the proportion of the compound represented by the formula (I) in the total amount of the compound represented by C₉F₁₈ may be from 10 mass% to 90 mass%, or from 30 mass% to 90 mass%, or from 10 mass% to 85 mass%, or from 35 mass% to 85 mass%, or not less than 10 mass% and less than 85 mass%, or not less than 20 mass% and less than 85 mass%, or not less than 30 mass% and less than 85 mass%, or not less than 40 mass% and less than 85 mass%, or not less than 50 mass% and less than 85 mass%, or from 40 mass% to 80 mass%, or from 55 mass% to 80 mass%, or from 60 mass% to 75 mass%, or from 65 mass% to 70 mass%, or from 35 mass% to 60 mass%, or from 50 mass% to 60 mass%, and it may be preferably from 30 mass% to 90 mass%, preferably not less than 40 mass% and less than 85 mass%, more preferably from 40 mass% to 80 mass%, further preferably from 45 mass% to 70 mass%, further more preferably from 50 mass% to 60 mass%.

Similarly, the proportion of the compound represented by the formula (II) in the total of the HFP trimer is preferably 1 mass% or more, more preferably 5 mass% or more, particularly preferably 10 mass% or more. Moreover, the compound represented by the formula (II) in the total of the HFP trimer is preferably 70 mass% or less, more preferably 50 mass% or less, yet further preferably 30 mass% or less, particularly preferably 20 mass% or less.

Similarly, the proportion of the compound represented by the formula (III) in the total of the HFP trimer is preferably 1 mass% or more, more preferably 5 mass% or more, particularly preferably 10 mass% or more. Moreover, the compound represented by the formula (III) in the total of the HFP trimer is preferably 70 mass% or less, more preferably 50 mass% or less, yet further preferably 30 mass% or less, particularly preferably 20 mass% or less.

The mass ratio between the compound represented by the formula (II) and the compound represented by the formula (III) in the HFP trimer is not limited, and, for example, it may be from 1:9 to 9: 1, or from 2:8 to 8:2, or from 3:7 to 7:3, or from 4:6 to 6:4, or from 4.5:5.5 to 5.5:4.5.

The content of the compound represented by C₉F₁₈ in the HFP trimer is preferably 20 mass% or more, more preferably 40 mass% or more, further preferably 60 mass% or more, particularly preferably 80 mass% or more, relative to the entire composition for heat transfer fluid. Moreover, the content of the compound represented by C₉F₁₈ contained in the composition for heat transfer fluid is preferably 99.9999 mass% or less relative to the entire composition for heat transfer fluid.

The mass ratio between the compound represented by the formula (II) and the compound represented by the formula (III) in the entire HFP trimer is not limited, and, for example, it may be from 1:9 to 9: 1, or from 2:8 to 8:2, or from 3:7 to 7:3, or from 4:6 to 6:4, or from 4.5:5.5 to 5.5:4.5.

The HFP trimer may include a hexafluoropropene trimer represented by C₉F₁₈ other than the compounds represented by the formulae (I) to (III).

The HFP trimer may include a hexafluoropropene dimer.

The hexafluoropropene dimer may include (E)-1,1,1,2,3,4,5,5,5-nonafluoro-4-(trifluoromethyl)-2-pentene, (Z)-1,1,1,2,3,4,5,5,5-nonafluoro-4-(trifluoromethyl)-2-pentene, or 1,1,3,4,4,5,5-nonafluoro-2-(trifluoromethyl)-2-pentene.

In one aspect, the HFP trimer may include a hexafluoropropene tetramer.

The hexafluoropropene tetramer may include 1,1,1,2,5,6,6,6-octafluoro-2,3,5-tris(trifluoromethyl)-4-(perfluoropropyl-2-yl)-3-hexene.

The compounds represented by the formulae (I) to (III) can be produced by a conventional procedure; for example, the compounds can be obtained by a method described in WO2018/172919, but this is not limitative, and the compounds can be obtained by a method selected from a wide range of known methods. Moreover, the compounds may be obtained by trimerization of HFPs.

The composition according to the present disclosure may contain an additional heat transfer fluid compound that is different from the compound represented by C₉F₁₈. The additional heat transfer fluid compound may be of one or more types. Examples of the additional heat transfer fluid include perfluorotripropylamine, perfluoropolyether, a methoxytridecafluoroheptene isomer mixture, perfluorotributylamine, and the like.

The perfluorotripropylamine is also referred to as tris(heptafluoropropyl)amine or N,N-bis(heptafluoropropyl)(heptafluoropropyl)amine, and represented by the general formula N(CF₂CF₂CF₃)ₙ(CF(CF₃)CF₃)₃₋ₙ (where n is an integer of 0 to 3). The perfluorotripropylamine may include only one type of compounds represented by the above general formula, or may include a plurality of types of them. N(CF₂CF₂CF₃)₃ is preferable, and N(CF₂CF₂CF₃)ₙ(CF(CF₃)CF₃)₃₋ₙ (where n is an integer of 0 to 2) may be included as impurities. Specific examples include a product under the trade name "Fluorinert (registered trademark)" (manufactured by 3M) (FC-3283) and the like.

The perfluoropolyether is preferably represented by the general formula:

RO-Rf¹-R'

where
R and R' are the same as or different from each other and each is a monovalent group represented by -CₘF₂ₘ₊₁, in which m is an integer of 1 to 8, and
Rf¹ is a divalent fluoropolyoxyalkylene group containing 2 to 20 repeating units, in which the repeating unit is represented by:
   (i) -CFXO- (where X is F or CF₃);
   (ii) -CF₂CFXO- (where X is F or CF₃);
   (iii) -CFXCF₂O- (where X is F or CF₃);
   (iv) -CF₂CF₂CF₂O-; or
   (v) -CF₂CF₂CF₂CF₂O-, or
Rf¹ is a divalent group represented by:
   (vi) -(CF₂)ₙ-CFY-O- (where n is an integer of 0 to 3, Y is a monovalent group represented by the general formula -ORf²Z, Rf² is a divalent fluoropolyoxyalkylene group containing 2 to 20 repeating units represented by - CFXO-, -CF₂CFXO-, -CF₂CF₂CF₂O-, or -CF₂CF₂CF₂CF₂O-, Xs are the same as or different from each other and each is F or CF₃, and Z is a monovalent C₁₋₅ perfluoroalkyl group).

Specific examples of the perfluoropolyether include products under the trade names GALDEN (registered trademark) "HT135" and GALDEN (registered trademark) "HT110" (both manufactured by Solvay), and the like.

Specifically, the methoxytridecafluoroheptene isomer mixture includes methyl-perfluoroheptene ether (MPHE) (C₇F₁₃OCH₃). Specific examples include a product under the trade name "Opteon SF10" (manufactured by Chemours) and the like.

The composition according to the present disclosure preferably contains the compound represented by C₉F₁₈ in 20 mass% or more, more preferably 40 mass% or more, further preferably 60 mass% or more, most preferably 80 mass% or more, relative to the entire heat transfer fluid. When perfluorotripropylamine, perfluoropolyether, and a methoxytridecafluoroheptene isomer mixture are contained, the content ratio of these components does not substantially affect the heat transfer fluid properties of the entire composition because these components have similar heat transfer fluid properties to those of the compound represented by C₉F₁₈. Therefore, in this case, the composition according to the present disclosure preferably contains the compound represented by C₉F₁₈ in 40 mass% to 99.9 mass%, more preferably 60 mass% to 99.9 mass%, further preferably 80 mass% to 99.9 mass%, relative to the entire heat transfer fluid.

### (2) Other Components

The composition according to the present disclosure may further contain other components in addition to the heat transfer fluid compound.

The composition according to the present disclosure may contain a stabilizer. The stabilizer exhibits stabilizing effects and thereby it functions as a so-called acid scavenger or antioxidant. Examples of major stabilizing effects include effects to capture radicals produced in the system to prevent degradation of the heat transfer fluid compound, acid scavenging effects to capture acids produced in the system to prevent further degradation of the heat transfer fluid compound due to acid, and the like.

The stabilizer may be selected from a wide range of known stabilizers. Among these, preferably, for effectively reducing metal corrosion caused by the composition, one or more types of stabilizers selected from the group consisting of unsaturated alcohol-based stabilizers, nitro-based stabilizers, amine-based stabilizers, phenol-based stabilizers, and epoxy-based stabilizers are used.

The unsaturated alcohol-based stabilizer may be selected from a wide range of known ones. For example, one or more selected from the group consisting of 3-buten-2-ol, 2-buten-1-ol, 4-propen-1-ol, 1-propen-3-ol, 2-methyl-3-buten-2-ol, 3-methyl-3-buten-2-ol, 3-methyl-2-buten-1-ol, 2-hexen-1-ol, 2,4-hexadien-1-ol, and oleyl alcohol can be used.

The nitro-based stabilizer may be selected from a wide range of known ones. As aliphatic nitro compounds, nitromethane, nitroethane, 1-nitropropane, 2-nitropropane, and the like may be mentioned, for example. As aromatic nitro compounds, one or more selected from the group consisting of nitrobenzene, o-, m-, and p-dinitrobenzenes, o-, m-, and p-nitrotoluenes, dimethylnitrobenzene, m-nitroacetophenone, o-, m-, and p-nitrophenols, o-nitroanisole, m-nitroanisole, and p-nitroanisole can be used, for example.

The amine-based stabilizer may be selected from a wide range of known ones. For example, one or more selected from the group consisting of pentylamine, hexylamine, diisopropylamine, diisobutylamine, di-n-propylamine, diallylamine, triethylamine, N-methylaniline, pyridine, morpholine, N-methylmorpholine, triallylamine, allylamine, α-methylbenzylamine, dimethylamine, trimethylamine, ethylamine, diethylamine, triethylamine, propylamine, isopropylamine, dipropylamine, tripropylamine, butylamine, isobutylamine, dibutylamine, tributylamine, dipentylamine, tripentylamine, 2-ethylhexylamine, aniline, N,N-dimethylaniline, N,N-diethylaniline, ethylenediamine, propylenediamine, diethylenetriamine, tetraethylenepentamine, benzylamine, dibenzylamine, diphenylamine, and diethylhydroxylamine can be used.

The phenol-based stabilizer may be selected from a wide range of known ones. For example, one or more selected from the group consisting of 2,6-di-tert-butyl-4-methylphenol, 3-cresol, phenol, 1,2-benzenediol, 2-isopropyl-5-methylphenol, and 2-methoxyphenol can be used.

The epoxy-based stabilizer may be selected from a wide range of known ones. For example, one or more selected from the group consisting of butylene oxide, 1,2-propylene oxide, 1,2-butylene oxide, butyl glycidyl ether, diethylene glycol diglycidyl ether, and 1,2-epoxy-3-phenoxypropane can be used.

Use of a combination of stabilizers with different stabilizing effects can more effectively prevent degradation of the heat transfer fluid compound that can occur from various causes, and therefore, it is preferable to use one or more selected from the group consisting of the epoxy-based stabilizers, the unsaturated alcohol-based stabilizers, the nitro-based stabilizers, and the phenol-based stabilizers mentioned above.

From the viewpoint of effectively reducing acid release from the heat transfer fluid compound to reduce metal corrosion caused by the liquid composition, the content ratio of the stabilizer in the entire composition is preferably 0.0001 mass% or more, more preferably 0.01 mass% or more. On the other hand, in consideration of avoiding undesired changes of the physical properties of the liquid composition due to excessive addition of stabilizers, the content ratio of the stabilizer in the entire composition is preferably 10 mass% or less, more preferably 5 mass% or less.

The composition according to the present disclosure may contain a compound represented by CₘF₂ₘ and/or CₙF₍₂ₙ₋₂₎ [where m is an integer of 4 to 12 except 9 and n is an integer of 4 to 12].

In the general formula, m is an integer of 4 or more, preferably an integer of 5 or more, more preferably an integer of 6 or more. Moreover, n is an integer of 12 or less, preferably an integer of 11 or less, more preferably an integer of 10 or less. It should be noted that m does not include 9.

In the general formula, n is an integer of 4 or more, preferably an integer of 5 or more, more preferably an integer of 6 or more. Moreover, n is an integer of 12 or less, preferably an integer of 11 or less, more preferably an integer of 10 or less. Particularly preferably, n is 9.

When the composition according to in the present disclosure contains CₘF₂ₘ and/or CₙF₍₂ₙ₋₂₎, stability of the compound represented by C₉F₁₈ is enhanced.

Preferably, the content of CₘF₂ₘ and/or CₙF₍₂ₙ₋₂₎ in the entire composition according to the present disclosure is 0.0001 mass% or more.

Moreover, the content of CₘF₂ₘ and/or CₙF₍₂ₙ₋₂₎ in the entire composition according to the present disclosure is preferably 10 mass% or less, preferably 5 mass% or less, further preferably 1 mass% or less.

In one aspect, in the composition according to the present disclosure, the content of CₘF₂ₘ and/or CₙF₍₂ₙ₋₂₎ relative to the total amount of the HFP trimer regarded as 100 parts by mass is preferably 0.0001 parts by mass or more, more preferably 0.01 parts by mass or more, further preferably 0.1 parts by mass or more.

Moreover, in the composition according to the present disclosure, the content of CₘF₂ₘ and/or CₙF₍₂ₙ₋₂₎ relative to the total amount of the HFP trimer regarded as 100 parts by mass is preferably 10 parts by mass or less, more preferably 5 parts by mass or less, further preferably 1 part by mass or less.

When multiple types of CₘF₂ₘ and/or CₙF₍₂ₙ₋₂₎ are contained, the above-mentioned content means the total amount of them.

When the content of CₘF₂ₘ and/or CₙF₍₂ₙ₋₂₎ falls within the above-mentioned range, degradation of the HFP trimer represented by C₉F₁₈ can be reduced and thereby an increase of fluoride ions and a rise of acidity can be reduced.

The composition according to the present disclosure may contain fluoride ions. When fluoride ions are contained, from the viewpoint of the thermal stability of the heat transfer fluid during long-term use, the content ratio in the entire composition is preferably from 0.0000001 to 5 mass%, more preferably from 0.000001 to 1 mass%.

The composition according to the present disclosure may contain water. When water is contained, from the viewpoint of the thermal stability of the heat transfer fluid during long-term use, the content ratio in the entire composition is preferably from 1 to 1000 ppm by mass.

In one aspect, the content of water relative to the total amount of the compound represented by C₉F₁₈ regarded as 100 parts by mass is preferably 0.0001 parts by mass or more (1 ppm or more), more preferably 0.0005 parts by mass or more (5 ppm or more), further preferably 0.001 parts by mass or more (10 ppm or more).

Moreover, the content of water relative to the total amount of the compound represented by C₉F₁₈ regarded as 100 parts by mass is preferably 10 parts by mass or less, more preferably 5 parts by mass or less, further preferably 1 part by mass or less.

The composition according to the present disclosure may contain conductive substances. Examples of the conductive substances include at least one selected from the group consisting of metals, metal ions, metal oxides, metal nitrides, carbon, conductive polymers, superconducting ceramic, and mixtures of these. When conductive substances are contained, from the viewpoint of reducing undesirable results such as a short circuit caused by the conductive substances entering into the device through a gap, the content ratio in the entire composition is preferably 10000 ppm by mass or less, more preferably 1000 ppm by mass or less, further preferably 1 ppm by mass or less.

The composition according to the present disclosure may contain insoluble matter. When insoluble matter is contained, from the viewpoint of inhibiting clogging of the piping and/or an increase of resistance in the circuit during repeated use, the content ratio in the entire composition is preferably 5000 ppm by mass or less, more preferably 1000 ppm by mass or less.

### 2. Use as Replacement for Heat Transfer Fluid

The composition according to the present disclosure is usable as a replacement composition for a heat transfer fluid containing at least one selected from the group consisting of perfluorotripropylamine, perfluoropolyether, and a methoxytridecafluoroheptene isomer mixture. The composition according to the present disclosure is usable as a replacement for currently-used heat transfer fluids listed below.
·Perfluorotripropylamine (or tris(heptafluoropropyl)amine, or N,N-bis(heptafluoropropyl)(heptafluoropropyl)amine) [N(CF₂CF₂CF₃)ₙ(CF(CF₃)CF₃)₃₋ₙ (where n is an integer of 0 to 3)], including a product under the trade name "Fluorinert (registered trademark)" (manufactured by 3M) (FC-3283);
·Perfluoropolyether (PFPE) including products under the trade names GALDEN (registered trademark) "HT135" and GALDEN (registered trademark) "HT110" (manufactured by Solvay), and specifically, compounds represented by the above-mentioned general formula RO-Rf-R', further more specifically tetrafluoroethylene oxide polymers and hexafluoropropylene oxides; and
·Methoxytridecafluoroheptene isomer mixtures including a product under the trade name "Opteon SF10" (manufactured by Chemours), and more specifically methyl-perfluoroheptene ether (MPHE) (C₇F₁₃OCH₃).

Specifically, within a unit designed to transfer heat by using the above-mentioned heat transfer fluid, the composition according to the present disclosure can be used as a replacement for the heat transfer fluid.

The composition according to the present disclosure can be a drop-in replacement, a nearly drop-in replacement, or a retrofit replacement for the above-mentioned heat transfer fluid. "A drop-in replacement" means a replacement without requiring any changes to the unit. "A nearly drop-in replacement" means a replacement requiring only few changes to the unit. "A retrofit replacement" means a replacement requiring minimal changes (namely, not requiring extensive changes) to the unit. Preferably, the composition according to the present disclosure can be a drop-in replacement or a nearly drop-in replacement for the above-mentioned heat transfer fluid.

Determination that use of the composition according to the present disclosure as a drop-in replacement, a nearly drop-in replacement, or a retrofit replacement is possible can be made when all the following conditions are satisfied.
(i) The boiling point of the composition according to the present disclosure is at least about 80% or more, preferably at least about 85% or more, of the boiling point of the predecessor heat transfer fluid.
(ii) The pour point of the composition according to the present disclosure is equal to or less than the pour point of the predecessor heat transfer fluid.
(iii) The kinematic viscosity of the composition according to the present disclosure is at least about 200% or less, preferably at least about 150% or less, of the kinematic viscosity of the predecessor heat transfer fluid.
(iv) The composition according to the present disclosure is compatible with the predecessor heat transfer fluid at a freely-selected proportion.

When the boiling point of the composition according to the present disclosure is designed to be at least about 80% or more, preferably at least about 85% or more, of the boiling point of the predecessor heat transfer fluid, occurrence of cavitation and leakage from the apparatus can be reduced. The upper limit to the boiling point of the heat transfer fluid is not limited, and, for example, it may be at least about 130% or less of the boiling point of the predecessor heat transfer fluid.

When the pour point of the composition according to the present disclosure is designed to be equal to or less than the pour point of the predecessor heat transfer fluid, use at a temperature less than the conventional operational temperature is possible and, thereby, the operational temperature range can be extended. The upper limit to the pour point of the heat transfer fluid is not limited, and, for example, it may be equal to or less than the temperature 30°C more than the pour point of the predecessor heat transfer fluid.

When the kinematic viscosity of the composition according to the present disclosure is designed to be at least about 200% or less, preferably at least about 150% or less, of the kinematic viscosity of the predecessor heat transfer fluid, an increase of power consumption can be reduced or power consumption can be reduced. Comparison of the kinematic viscosity is preferably performed in terms of the kinematic viscosity at the operational temperature, but this is not limitative, and the comparison can be performed in terms of the kinematic viscosity at a temperature from -20°C to -40°C, for example, and more specifically, it can be performed at -20°C.

When the composition according to the present disclosure is compatible with the predecessor heat transfer fluid at a freely-selected proportion, replacing operation is easier to carry out.

Furthermore, when the following conditions are satisfied, the composition according to the present disclosure is more suitable as a drop-in replacement, a nearly drop-in replacement, or a retrofit replacement.
(v) The composition according to the present disclosure has a dielectric constant that is 120% or less of that of the predecessor heat transfer fluid.
(vi) The composition according to the present disclosure has a dielectric strength that is 90% or more of that of the predecessor heat transfer fluid.
(vii) The composition according to the present disclosure has a specific heat that is 90% or more of that of the predecessor heat transfer fluid.
(viii) The composition according to the present disclosure has a thermal conductivity that is 85% or more of that of the predecessor heat transfer fluid.

When having a dielectric constant designed to be 120% or less of the dielectric constant of the predecessor heat transfer fluid, the composition according to the present disclosure can be suitably used as a replacement composition. The lower limit to the dielectric constant of the heat transfer fluid is not limited, and, for example, it may be 80% or more of the dielectric constant of the predecessor heat transfer fluid.

When having a dielectric strength designed to be 90% or more of the dielectric strength of the predecessor heat transfer fluid, the composition according to the present disclosure can be suitably used as a replacement composition. The upper limit to the dielectric strength of the heat transfer fluid is not limited, and, for example, it may be 120% or less of the dielectric strength of the predecessor heat transfer fluid.

When having a specific heat designed to be 90% or more of the specific heat of the predecessor heat transfer fluid, the composition according to the present disclosure can be suitably used as a replacement composition. The upper limit to the specific heat of the heat transfer fluid is not limited, and, for example, it may be 120% or less of the specific heat of the predecessor heat transfer fluid.

When having a thermal conductivity designed to be 85% or more of the thermal conductivity of the predecessor heat transfer fluid, the composition according to the present disclosure can be suitably used as a replacement composition. The upper limit to the thermal conductivity of the heat transfer fluid is not limited, and, for example, it may be 120% or less of the thermal conductivity of the predecessor heat transfer fluid.

The boiling point of the composition according to the present disclosure may be preferably 105°C or more, more preferably 108°C or more. The upper limit to the boiling point of the composition according to the present disclosure is not limited, and, for example, it may be 150°C or less, or 130°C or less, or 120°C or less.

The pour point of the composition according to the present disclosure may be preferably -80°C or less, more preferably -100°C or less, further preferably -110°C or less. The lower limit to the pour point of the composition according to the present disclosure is not limited, and, for example, it may be -180°C or more, or -160°C or more.

The kinematic viscosity of the composition according to the present disclosure at -20°C may be preferably 6.0 cSt or less, more preferably 5.0 cSt or less, further preferably 4.0 cSt or less, further more preferably 3.5 cSt or less. The lower limit to the kinematic viscosity of the composition according to the present disclosure is not limited, and, for example, it may be 1.0 cSt or more.

The dielectric constant of the composition according to the present disclosure may be preferably 3.0 or less, more preferably 2.5 or less, further preferably 2.0 or less. The lower limit to the dielectric constant of the composition according to the present disclosure is not limited, and, for example, it may be 1.1 or more.

The dielectric strength of the composition according to the present disclosure may be preferably 40 kV or more, more preferably 50 kV or more. The upper limit to the dielectric strength of the composition according to the present disclosure is not limited, and, for example, it may be 150 kV or less, or 100 kV or less.

The specific heat of the composition according to the present disclosure at 30°C may be preferably 800 J/kg·K or more, more preferably 900 J/kg·K or more, further preferably 1000 J/kg·K or more. The upper limit to the specific heat of the composition according to the present disclosure is not limited, and, for example, it may be 2000 J/kg·K or less, or 1500 J/kg·K or less.

The thermal conductivity of the composition according to the present disclosure at 30°C may be preferably 0.055 W/mK or more, more preferably 0.060 W/mK or more, further preferably 0.065 W/mK or more. The upper limit to the thermal conductivity of the composition according to the present disclosure is not limited, and, for example, it may be 0.090 W/mK or less, or 0.080 W/mK or less.

The boiling point of the composition according to the present disclosure is measured by DSC (differential scanning calorimetry), as a temperature at which an endothermic peak is observed while the temperature is being raised from 25°C at 5°C/minute.

The pour point of the composition according to the present disclosure is measured by DSC, as a temperature at which an endothermic peak is observed while the temperature is being raised at 5°C/minute after lowered with liquid nitrogen to the coagulation point or less.

The dielectric constant of the composition according to the present disclosure is a value at a frequency of 1 kHz measured by a capacitance method in an environment at a temperature of 25°C and a humidity of 60%.

Each of the kinematic viscosity and the density of the composition according to the present disclosure is a value measured with a kinematic viscometer, SVM3001, manufactured by Anton Paar.

The dielectric strength of the composition according to the present disclosure is the breakdown voltage measured at the time when a liquid specimen is made to enter into the gaps between spherical electrodes aligned at regular intervals and then the voltage is raised at a steady rate. Measurement conditions are as described below.
Electrode shape: Spherical (φ12.5 mm)
Intervals between electrodes: 2.5 mm
Pressure-raising rate: 2 kV/second
Atmosphere of measurement: Air (22°C, 57%RH)

The specific heat of the composition according to the present disclosure is a value obtained by DSC under conditions described below.
Measurement apparatus: Differential scanning calorimeter DSC8500 manufactured by Perkin-Elmer
Temperature raising rate: 10°C/minute
Reference specimen: Sapphire (-Al2O3)
Atmosphere: In dry nitrogen stream
Specimen container: Hermetically sealed aluminum container

The thermal conductivity of the composition according to the present disclosure is a value obtained by a transient hot-wire method.

The compatibility of the composition according to the present disclosure is determined by checking if it is compatible with a target solvent when mixed. Herein, "being compatible" means that the two are mixed uniformly, in other words, phase separation does not occur.

### 3. Unit Comprising Heat Transfer Fluid

A unit according to the present disclosure comprises the composition according to the present disclosure.

The unit according to the present disclosure comprises a device that comprises a device and a heat transfer mechanism for transferring heat to the device or from the device.

Specifically, the device according to the present disclosure is a device comprising a heat transfer mechanism, wherein the device comprises a device and a heat transfer mechanism that transfers heat to the device or from the device by using the composition according to the present disclosure as a heat transfer fluid, and the heat transfer mechanism is designed to transfer heat by using a heat transfer fluid containing at least one selected from the group consisting of perfluorotripropylamine, perfluoropolyether, and a methoxytridecafluoroheptene isomer mixture.

Examples of the device include computers, server computers, servers including blade servers; disk array/storage systems; storage area networks; network-attached storages; storage communications systems; workstations; routers; telecommunications infrastructures/switches; wired, optical, and wireless communications apparatuses; cell processing apparatuses; printers; power source apparatuses; displays; optical apparatuses; measurement systems including hand-held systems; military electronic devices; and the like.

Semiconductor devices are heat-generating devices to be mounted on the device, such as CPUs, GPUs, SSDs, and the like, for example, and a semiconductor device is made of a single element such as silicon and germanium, a compound semiconductor such as gallium arsenide (GaAs), gallium phosphide (GaP), indium phosphide (InP), gallium nitride (GaN), and silicon carbide (SiC), and/or the like, for example.

When the device is a server computer, one logic board or a plurality of logic boards are accommodated in the internal space. The logic board comprises many heat-generating electronic components including at least one processor such as CPU and/or GPU. In addition to them, other heat-generating components for computers such as those listed below, for example, can be used: chipsets; memories, graphics chips, network chips, RAMs, power source apparatuses, daughter cards; and storage drives such as solid state drives and mechanical hard drives.

The heat transfer mechanism is a heat transfer mechanism that transfers heat to and from a heat transfer target by using the composition according to the present disclosure, and the supplying and receiving heat (heat transfer) is performed through thermal contact with the heat transfer target. For example, drawing heat away from the heat transfer target is cooling, and supplying heat is heating. Different mechanisms may be used for cooling and heating, respectively, or a singe heat transfer mechanism may be used for both cooling and heating.

The heat transfer mechanism is not limited and, for example, it may be a pump, a valve, a fluid containment system, a pressure control system, a cooler, a heat exchanger, a heat source, a heat sink, a refrigeration system, an active temperature control system, a passive temperature control system, and/or the like.

More specific examples include a temperature-controlled wafer chuck installed inside a plasma enhanced chemical vapor deposition (PECVD) tool, a temperature-controlled test head for die performance testing, a temperature-controlled workspace inside a semiconductor process device, a reservoir for thermal shock test bath liquid, a thermostatic chamber, and the like.

Examples of the heat transfer target to be brought into thermal contact with the heat transfer mechanism include articles, apparatuses, and atmospheres that are cooled to, heated to, or maintained at an intended temperature. Examples of the heat transfer target include electric components, machine components, and optical components, as well as those after processing and those after assembly. Specific, non-limiting examples of the heat transfer target according to the present disclosure include microprocessors, wafers used for producing semiconductor devices, power control semiconductors, electric branch switches, power transformers, circuit boards, multichip modules, mounted and non-mounted semiconductor devices, chemical reactors, nuclear reactors, fuel cells, heat exchangers, electrochemical cells, lasers, missile components, and the like.

During the use of the unit according to the present disclosure, the temperature conditions of the composition according to the present disclosure are preferably from - 100 to 100°C, more preferably from -90 to 90°C, more preferably from -70 to 90°C. Advantageously, the composition according to the present disclosure exhibits a low kinematic viscosity at low temperatures of -20°C or less, even at -70 to -60°C, and therefore, the apparatus can be suitably used at temperatures within this range.

The unit according to the present disclosure further contains two-phase immersion cooling fluid, chiller fluid, Rankine cycle working fluid, and the like.

### 4. Heat Transfer Method

A heat transfer method according to the present disclosure is a heat transfer method implementable in a unit designed to transfer heat by using a heat transfer fluid containing at least one selected from the group consisting of perfluorotripropylamine, perfluoropolyether, and a methoxytridecafluoroheptene isomer mixture, where the heat transfer method comprises transferring heat by using the composition according to the present disclosure as a replacement for the heat transfer fluid.

The unit includes a device comprising a device and a heat transfer mechanism that transfers heat to the device or from the device, two-phase immersion cooling fluid, chiller fluid, Rankine cycle working fluid, and the like.

In regard to the above description, the device includes the device described in "3. Unit Comprising Heat Transfer Fluid". When the device is used, the heat transfer method according to the present disclosure is a heat transfer method implementable in a device comprising a heat transfer mechanism designed to transfer heat by using a heat transfer fluid containing at least one selected from the group consisting of perfluorotripropylamine, perfluoropolyether, and a methoxytridecafluoroheptene isomer mixture, where the heat transfer method comprises transferring heat to the device or from the device by operating the heat transfer mechanism with the use of the composition according to the present disclosure as a replacement for the heat transfer fluid.

In the heat transfer method according to the present disclosure mentioned above, the heat transfer mechanism is placed in thermal contact with the device so as to make heat transfer possible. When placed in thermal contact with the device, the heat transfer mechanism can draw heat away from the device, or supply heat to the device, or maintain the device at a selected temperature or within a selected temperature range. The direction of heat flow (from the device or to the device) depends on the relative temperature difference between the device and the heat transfer mechanism.

Thus far, embodiments of the present invention are described, but it is obvious that the present invention is by no means limited to those examples and can be implemented in various configurations without departing from the gist of the present invention.

### Examples

In the following, a more detailed description will be given of embodiments of the present invention by way of Examples, but these Examples are not intended to limit the scope of the present invention.

### <Example 1>

Based on a method described in Chem Ber 1973, 106, 2950-2959, a composition for heat transfer fluid of Example 1 was obtained. The HFP trimer thus obtained was purified by distillation for removal of impurities such as hexafluoropropene dimers and tetramers. The purified HFP trimer was distillated and thereby separated into compounds represented by the formulae (I), (II), and (III). Each HFP trimer thus separated was dehydrated on silica gel.

The compounds represented by the formulae (I), (II), and (III) thus obtained were mixed in such a manner that the ratio between the compounds represented by the formulae (I), (II), and (III) became as specified in the table below, and thereby trimer mixtures of Examples 1 to 4 were obtained.

**[Table 1]**

| Example | Formula (I) | Formula (II) | Formula (III) |
|---|---|---|---|
| 1 | 1.5 mass% | 35.5 mass% | 63.0 mass% |
| 2 | 52.0 mass% | 16.0 mass% | 32.0 mass% |
| 3 | 77.0 mass% | 8.0 mass% | 15.0 mass% |
| 4 | 90.0 mass% | 5.0 mass% | 5.0 mass% |

### <Comparative Example 1>

For Comparative Example 1, FC-3283 manufactured by 3M was prepared. This did not contain compounds represented by (I), (II), and (III).

### <Comparative Example 2>

For Comparative Example 2, HFE7100 manufactured by 3M was prepared. This did not contain compounds represented by (I), (II), and (III).

The compositions of Examples 1 to 4 and Comparative Examples 1 to 2 were subjected to evaluation of the boiling point, pour point, dielectric constant, kinematic viscosity, dielectric strength, specific heat, thermal conductivity, and compatibility.

### <Measurement of Boiling Point, Pour Point, and Dielectric Constant>

The boiling point of the trimer mixture was measured by DSC (differential scanning calorimetry), as a temperature at which an endothermic peak was observed while the temperature was being raised from 25°C at 5°C/min. The pour point was measured by DSC, as a temperature at which an endothermic peak was observed while the temperature was being raised at 5°C/min after lowered with liquid nitrogen to the coagulation point or less. The dielectric constant was a value at a frequency of 1 kHz measured by a capacitance method in an environment at a temperature of 25°C and a humidity of 60%.

### <Measurement of Kinematic Viscosity and Density>

The kinematic viscosity and the density of the trimer mixture were measured with a kinematic viscometer, SVM3001, manufactured by Anton Paar.

### <Measurement of Dielectric Strength>

A liquid specimen was made to enter into the gaps between spherical electrodes aligned at regular intervals, followed by raising the voltage at a steady rate, and the breakdown voltage was measured and regarded as the dielectric strength of the trimer mixture. Specific conditions of the measurement were as described below.
Electrode shape: Spherical (φ12.5 mm)
Intervals between electrodes: 2.5 mm
Pressure-raising rate: 2 kV/second
Atmosphere of measurement: Air (22°C, 57%RH)

### <Measurement of Specific Heat>

The specific heat of the trimer mixture was measured by DSC. The conditions of the measurement were as described below.
Measurement apparatus: Differential scanning calorimeter DSC8500 manufactured by Perkin-Elmer
Temperature raising rate: 10°C/minute
Reference specimen: Sapphire (-Al2O3)
Atmosphere: In dry nitrogen stream
Specimen container: Hermetically sealed aluminum container

### <Measurement of Thermal Conductivity>

The thermal conductivity of the trimer mixture was measured by a transient hot-wire method.

### <Compatibility>

The compatibility of the trimer mixture was evaluated by using a mixture of equal amounts of the below-listed three types of solvents and each trimer mixture.
Galden HT135 (manufactured by Solvay)
SF-10 (manufactured by Chemours)
FC3283 (manufactured by 3M)

**[Table 2]**

| | Example 1 | Example 2 | Example 3 | Example 4 | Comp. Ex. 1 | Comp. Ex. 2 |
|---|---|---|---|---|---|---|
| Boiling point (°C) | 112 | 109 | 109 | 108.5 | 128 | 61 |
| Pour point (°C) | -110 | <-130 | <-130 | <-130 | -65 | <-130 |
| Dielectric constant (1 kHz) | 1.97 | 2.00 | 2.00 | 2.00 | 1.9 | 7.5 |
| Kinematic viscosity (cSt) @-20°C | 4.8 | 3.4 | 2.5 | 2.4 | 2.4 | -- |
| Dielectric strength (kV) | >40 | >40 | >40 | >40 | >40 | 28 |
| Specific heat (J/kg·K) @30°C | 1100 | 1060 | 1060 | 1060 | 1040 | 1170 |
| Thermal conductivity (W/mK) @30°C | 0.074 | 0.064 | 0.062 | 0.060 | 0.064 | 0.069 |
| Compatibility | Compatible with 3 types | Compatible with 3 types | Compatible with 3 types | Compatible with 3 types | -- | -- |

## Claims

1. A composition containing a hexafluoropropene trimer represented by C₉F₁₈, wherein the composition is usable as a replacement for a heat transfer fluid containing at least one selected from the group consisting of perfluorotripropylamine, perfluoropolyether, and a methoxytridecafluoroheptene isomer mixture.

2. The composition according to claim 1, wherein the hexafluoropropene trimer represented by C₉F₁₈ includes at least one compound selected from the group consisting of compounds represented by formulae (I) to (III) below.

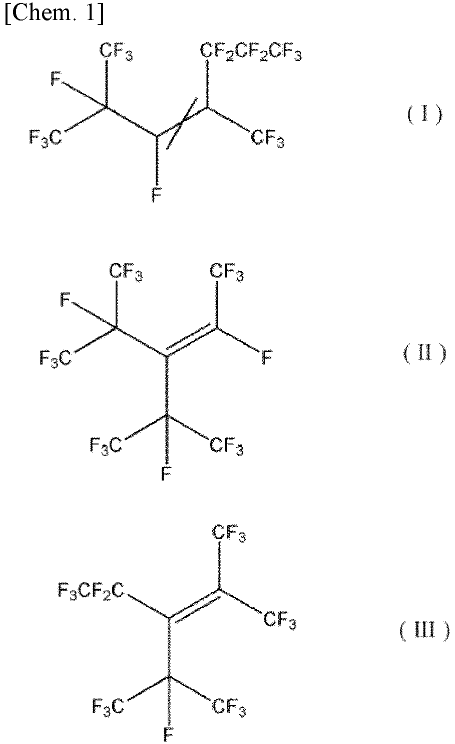

3. The composition according to claim 2, wherein the hexafluoropropene trimer represented by C₉F₁₈ includes the compound represented by the formula (I) in less than 85 mass% relative to a total of the hexafluoropropene trimer.

4. The composition according to any one of claims 1 to 3, wherein the composition satisfies conditions (i) to (vi) below:
(i) having a boiling point that is 80% or more of a boiling point of the heat transfer fluid;
(ii) having a pour point that is equal to or less than a pour point of the heat transfer fluid;
(iii) having a kinematic viscosity that is 200% or less of a kinematic viscosity of the heat transfer fluid; and
(iv) being compatible with the heat transfer fluid at a freely-selected proportion.

5. A composition for heat transfer fluid, comprising:
a hexafluoropropene trimer represented by C₉F₁₈; and
at least one selected from the group consisting of perfluorotripropylamine, perfluoropolyether, and a methoxytridecafluoroheptene isomer mixture.

6. The composition for heat transfer fluid according to claim 5, wherein the hexafluoropropene trimer represented by C₉F₁₈ includes at least one compound selected from the group consisting of compounds represented by formulae (I) to (III) below.

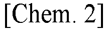

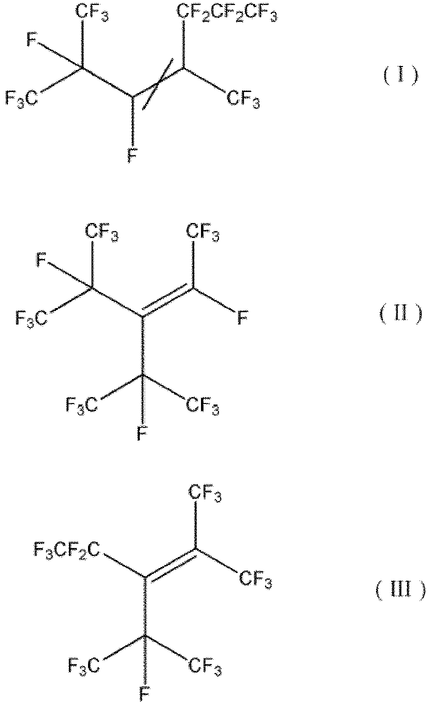

7. The composition for heat transfer fluid according to claim 6, wherein the compound includes the compound represented by the formula (I) in less than 85 mass% relative to a total of the hexafluoropropene trimer represented by C₉F₁₈.

8. The composition for heat transfer fluid according to claim 6 or 7, wherein the compound includes the compound represented by the formula (I) in 85 mass% or more relative to a total of the hexafluoropropene trimer represented by C₉F₁₈.

9. A device comprising:
a device; and
a heat transfer mechanism that transfers heat to the device or from the device by using the composition according to any one of claims 1 to 4 or the composition for heat transfer fluid according to any one of claims 5 to 8 as a heat transfer fluid,
wherein the heat transfer mechanism is designed to transfer heat by using a heat transfer fluid containing at least one selected from the group consisting of perfluorotripropylamine, perfluoropolyether, and a methoxytridecafluoroheptene isomer mixture.

10. Use of a hexafluoropropene trimer represented by C₉F₁₈ as a replacement for a heat transfer fluid containing at least one selected from the group consisting of perfluorotripropylamine, perfluoropolyether, and a methoxytridecafluoroheptene isomer mixture.

11. A heat transfer method implementable in a device comprising a heat transfer mechanism designed to transfer heat by using a heat transfer fluid containing at least one selected from the group consisting of perfluorotripropylamine, perfluoropolyether, and a methoxytridecafluoroheptene isomer mixture, the heat transfer method comprising:
transferring heat to the device or from the device by operating the heat transfer mechanism with the use of the composition according to any one of claims 1 to 4 or the composition for heat transfer fluid according to any one of claims 5 to 8 as a replacement for the heat transfer fluid.
